Europäisches Patentamt

European Patent Office

Office européen des brevets

(19)

(11) Publication number: **0 393 909**

**A1**

(12) # EUROPEAN PATENT APPLICATION

(21) Application number: **90303848.7**

(22) Date of filing: **10.04.90**

(51) Int. Cl.5: **A61K 33/14, A61K 31/70, A61K 31/445, //(A61K33/14, 33:00,31:70,31:445,31:195, 31:19),(A61K31/445,31:195, 31:19)**

(30) Priority: **13.04.89 GB 8908361**

(43) Date of publication of application:
**24.10.90 Bulletin 90/43**

(84) Designated Contracting States:
**AT BE CH DE DK ES FR GB GR IT LI LU NL SE**

(71) Applicant: **Beecham Group p.l.c.**
**SB House Great West Road**
**Brentford Middlesex TW8 9BD(GB)**

(72) Inventor: **James, Kenneth William, Beecham Products Research**
**Centre, St. George's Avenue**
**Weybridge, Surrey KT13 0DE(GB)**
Inventor: **Lea, Philip, Beecham Products Research**
**Centre, St. George's Avenue**
**Weybridge, Surrey KT13 0DE(GB)**

(74) Representative: **Dayneswood, Trevor et al**
**SmithKline Beecham, Corporate Patents,**
**Great Burgh, Yew Tree Bottom Road**
**Epsom Surrey KT18 5XQ(GB)**

(54) **Composition.**

(57) A pharmaceutical composition (preferably in reconstitutable form) comprising loperamide or a pharmaceutically acceptable salt or pro-drug thereof (e.g. its hydrochloride acid addition salt) and an oral rehydration agent such as a sugar, an oral rehydration salt, or an amino acid (especially a mixture of glucose, one or more chloride salts, and a bicarbonate or a citrate salt) in admixture or conjunction with a pharmaceutically acceptable carrier or excipient is useful in the treatment of diarrhoea. The loperamide may be used in 0.5 to 10 mg per unit dose, and the oral rehydration agent in 0.1 to 10 g per unit dose.

EP 0 393 909 A1

## COMPOSITION

The present invention relates to a novel drug combination useful in the treatment of diarrhoea.

The present invention provides a pharmaceutical composition comprising loperamide or a pharmaceutically acceptable salt or pro-drug thereof and an oral rehydration agent in admixture or conjunction with a pharmaceutically acceptable carrier or excipient.

Loperamide is the compound 4-(4-chlorophenyl)-4-hydroxy-N,N-dimethyl-α,α-diphenyl-1-piperidine-butanamide and is known to possess anti-diarrhoeal properties; more specifically, it has both anti-motility and anti-secretory action in the intestine, both assisting in the treatment of diarrhoea. It may be used in the form of the parent compound, in the form of a pharmaceutically acceptable acid addition salt, or in the form of a pro-drug.

Loperamide is commonly used in the form of its hydrochloride acid addition salt.

The term "pro-drug" as used herein means any derivative of the parent compound which will break down in vivo, i.e. in the human body, or, in other words, which is metabolisable, to give the desired parent compound or a salt thereof.

The term "loperamide" as used hereinafter includes the parent compound and its pharmaceutically acceptable acid addition salts and pro-drugs, except where the context obviously requires otherwise. All weights, ratios, dosages and the like specified herein are calculated on the respective parent compound.

According to the present invention, loperamide is used in combination with an oral rehydration agent in the treatment of diarrhoea in order both to stop the diarrhoea and to give overall improvement in patient well-being beyond that associated with conventional treatment using an anti-diarrhoeal agent or oral rehydration agent alone.

Oral rehydration agents assist in rehydration of the body following the substantial fluid loss often associated with diarrhoea. Oral rehydration agents which may be used in accordance with the present invention include those known to be suitable for this purpose and those used for this purpose in known rehydration formulations whether or not used in conjunction with an anti-diarrhoea agent.

Examples of suitable oral rehydration agents include sugars, oral rehydration salts, and amino acids, as well as mixtures of any two or more thereof.

Examples of oral rehydration salts include bicarbonate, citrate, and chloride salts. The salts may suitably be alkali metal salts, for example sodium or potassium salts, or alkaline earth metal salts, for example calcium or magnesium salts. The salts used should normally be water-soluble salts.

Individual examples of suitable oral rehydration salts include sodium bicarbonate, sodium citrate, sodium dihydrogen citrate, disodium hydrogen citrate, sodium chloride and potassium chloride.

In some cases, the oral rehydration salts may be formed in situ and the term "oral rehydration agent" as used herein encompasses products or product mixtures that will form an oral rehydration salt in situ, that is to say, for example, either when admixed with water immediately prior to administration to a patient or when admixed with body fluids within the patient. An example of such a product is a mixture of citric acid and sodium bicarbonate, which will react in solution to give sodium citrate.

Examples of sugars suitable for use as oral rehydration agents include sucrose, fructose, and glucose (suitably anhydrous glucose, glucose monohydrate, and including glucose polymers).

Examples of amino acids suitable for use as oral rehydration agents include alanine and glycine.

Advantageously, a composition according to the present invention includes a mixture of oral rehydration agents, comprising glucose, one or more chloride salts, and a bicarbonate or a citrate salt.

The oral rehydration agents may suitably be used in amounts of from 1 to 10 g per unit dose. Typically, glucose may be used in an amount of from 1 to 6 g per unit dose, with the oral rehydration salts being used in amounts of from 0.1 to 4 g per unit dose.

The loperamide may suitably to administered in a unit dose of from 0.5 to 10 mg per unit dose, preferably from 1 to 4 mg per unit dose.

(All unit dosages specified herein refer to those for an adult human, of approximately 70 kg body weight.)

The composition according to the invention may be formulated in any form adapted for oral administration to humans, but is most suitably formulated as a reconstitutable formulation, i.e. as a dry product for reconstitution with water. The composition according to the invention may most suitably be presented in the form of a reconstitutable powder (for example a non-effervescent or effervescent powder) in a sachet or the like, each sachet or the like containing a unit dosage, or in the form of effervescent tablets, where one or more tablets constitute a unit dosage.

The compositions according to the invention may contain conventional pharmaceutically acceptable materials, including, for example, diluents, binders, fillers, excipients, colours, flavours, preservatives, disintegrants, flow aids, lubricants, and wetting agents.

Compositions according to the invention presented in reconstitutable form may, in particular, contain conventional pharmaceutically acceptable auxiliaries selected from colours, flavours, flow aids (for example, colloidal silica), and wetting agents (for example, sodium lauryl sulphate or magnesium lauryl sulphate).

Effervescent powders or tablets may, for example, comprise citric acid and sodium bicarbonate to provide both the effervescence and the oral rehydration salts.

The compositions may contain from 1 to 99% by weight of the active ingredients, namely loperamide and the oral rehydration agents, preferably from 50 to 99%, and especially from 75 to 99%, by weight.

In general, the compositions according to the present invention may be formulated and manufactured in a conventional manner according to normal pharmaceutical practice.

When the compositions according to the invention are presented in reconstitutable form, for example as a reconstitutable powder in a unit-dosage sachet or the like or as effervescent tablets, the amount of water recommended to be used for reconstitution of the composition will depend largely on the amount of the oral rehydration agent(s) in the composition. As a practical matter, the amount of water for reconstitution of a unit dose should preferably be from 100 to 500 ml, especially from 200 to 300 ml (0.33 to 0.50 Imperial (U.K.) pint).

The following examples illustrates a composition according to the present invention.

Example 1

The following components:

| loperamide hydrochloride | 2.0 mg |
|---|---|
| anhydrous glucose | 3.00 g |
| sodium citrate | 0.44 g |
| sodium chloride | 0.26 g |
| potassium chloride | 0.23 g |
| flavouring | 0.04 g |
| colouring | 4.0 mg |
| saccharin sodium | 6.0 mg |
| colloidal silica | 7.5 mg |
| sodium lauryl sulphate | 1.0 mg |

are mixed together in a conventional manner and the resulting powder mixture is filled into a unit-dosage sachet.

The contents of the sachet may be dissolved in about 150-300 ml water immediately prior to consumption by a patient suffering from diarrhoea.

Example 2

The following components were formulated as described below:

| Premix | mg/dose |
|---|---|
| glucose anhydrous | 2500 |
| loperammide HCl | 2 |
| isopropyl alcohol | (52)* |
| deionised water | (200)* |
| deionised water Q.S. | (62)* |
| Final blend | |
| glucose anhydrous | 2500 |
| potassium chloride | 383 |
| sodium chloride | 433 |
| trisodium citrate | 733 |
| flavouring agents | 490 |

\* materials evaporated during process

The loperamide hydrochloride was added to the water and isopropyl alcohol with mixing, and heated to 55°C. The resulting loperamide solution was added slowly to the premix glucose with slow mixing. Additional water was added as required to give uniform mix dampness, and the mixture was stirred for about 5 minutes. The resulting mixture was wet-sieved, dried, and sieved.

Half the amount of the final blend glucose (predried at 70°C to give not more than 0.1% loss on drying), was added to a mixer. The flavouring agents were mixed with an equal amount of the glucose, sieved, and then added to the mixer. The oral rehydration salts were sieved, mixed with the remaining glucose, and with the loperamide premix, and added to the mixer.

The final mix was filled into one-dose sachets, each of which may be dissolved in about 250 ml water immediately prior to consumption.

**Claims**

1. A pharmaceutical composition comprising loperamide or a pharmaceutically acceptable salt or pro-drug thereof and an oral rehydration agent in admixture or conjunction with a pharmaceutically acceptable carrier or excipient.

2. A composition as claimed in claim 1, wherein the loperamide is used in the form of its hydrochloride acid addition salt.

3. A composition as claimed in claim 1 or claim 2, wherein the oral rehydration agent comprises a sugar, an oral rehydration salt, or an amino acid.

4. A composition as claimed in claim 3, wherein the oral rehydration salt is an alkali metal bicarbonate, citrate, or chloride salt.

5. A composition as claimed in claim 3 or claim 4, wherein the oral rehydration salt is formed in situ.

6. A composition as claimed in any one of claims 3 to 5, wherein the oral rehydration agent comprises sucrose, fructose, glucose, alanine, glycine, or a combination of any two or more thereof.

7. A composition as claimed in claim 1 or claim 2, which includes a mixture of oral rehydration agents, comprising glucose, one or more chloride salts, and a bicarbonate or a citrate salt.

8. A composition as claimed in any one of claims 1 to 7, wherein the oral rehydration agent is present in an amount of from 0.1 to 10 g per unit dose.

9. A composition as claimed in any one of claims 1 to 8, wherein the loperamide is present in an amount of from 0.5 to 10 mg per unit dose.

10. A composition as claimed in any one of claims 1 to 9, which is formulated in a reconstitutable form.

11. A composition as claimed in claim 1, substantially as described in the example herein.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 112 061  (BEECHAM GROUP PLC)<br>* Page 11, formulation 5; pages 1-2, claims 1-10 *<br>----- | 1-11 | A 61 K  33/14<br>A 61 K  31/70<br>A 61 K  31/445 //<br>(A 61 K  33/14<br>A 61 K  33:00<br>A 61 K  31:70<br>A 61 K  31:445<br>A 61 K  31:195<br>A 61 K  31:19 )<br>(A 61 K  31/445<br>A 61 K  31:195<br>A 61 K  31:19 ) |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 26-07-1990 | BRINKMANN C. |